Europäisches Patentamt

European Patent Office

Office européen des brevets

(19)

(11) Publication number: **0 275 689 B1**

## EUROPEAN PATENT SPECIFICATION

(12)

(45) Date of publication of patent specification: **02.09.92**

(51) Int. Cl.5: **C12N 15/31**, A61K 39/10, C12N 15/70

(21) Application number: **87311279.1**

(22) Date of filing: **22.12.87**

The file contains technical information submitted after the application was filed and not included in this specification

(54) **Modified pertussis exotoxin.**

(30) Priority: **23.12.86 US 945832**

(43) Date of publication of application:
**27.07.88 Bulletin 88/30**

(45) Publication of the grant of the patent:
**02.09.92 Bulletin 92/36**

(84) Designated Contracting States:
**AT BE CH DE ES FR GB GR IT LI LU NL SE**

(56) References cited:
**EP-A- 0 087 735**
**EP-A- 0 145 486**
**EP-A- 0 232 229**
**EP-A- 0 235 474**

**GENE, vol. 50, 1986, pages 133-140, Elsevier Science Publishers B.V. (Biomedical Division); S. STIBITZ et al.: "The construction of a cloning vector designed for gene replacement in Bordetella pertussis"**

(73) Proprietor: **THE BOARD OF TRUSTEES OF THE LELAND STANFORD JUNIOR UNIVERSITY**
**Encina 105 Stanford University**
**Stanford California 94305(US)**

(72) Inventor: **Black, William J.**
**890 Fielding Avenue**
**Palo Alto California 94303(US)**
Inventor: **Falkow, Stanley**
**8 Longspur**
**Portola Valley California 94025(US)**

(74) Representative: **Harrison, David Christopher et al**
**MEWBURN ELLIS & CO 2/3 Cursitor Street London EC4A 1BO(GB)**

EP 0 275 689 B1

BIOCHEMISTRY, vol. 21, 1982, pages 5516-5522, American Chemical Society; M. TAMURA et al.: "Subunit structure of islet-activating protein, pertussis toxin, in conformity with the A-B mode"

CHEMICAL ABSTRACTS, vol. 105, no. 19, 10th November 1986, page 199, abstract no. 166239w, Columbus, Ohio, US; J. REISER et al.: "Expression in Escherichia coli of a Bordetella pertussis toxin-coding region", & VACCINES 86, NEW APPROACHES IMMUN., [PROC. CONF.] 1985 (PUB. 1986), 235-8

SCIENCE, vol. 232, 6th June 1986, pages 1258-1264; C. LOCHT et al.: "Pertussis toxin gene: nucleotide sequence and genetic organization"

CHEMICAL ABSTRACTS, vol. 106, no. 11, 16th March 1987, page 163, abstract no. 79657h, C0olumbus, Ohio, US; J.M. KEITH et al.: "Pertussis toxin gene cloning and expression of protective antigen", & US-A-843 727 (UNITED STATES DEPT. OF HEALTH AND HUMAN SERVICES) 20-06-1986

CHEMICAL ABSTRACTS, vol. 106, no. 5, 2nd February 1987, page 120, abstract no. 28346y, Columbus, Ohio, US; R.M. BROWNLIE et al.: "Complementation of mutations in Escherichia coli and Bordetella pertussis by B. pertussis DNA cloned in a broad-host-range cosmid vector", & J. GEN. MICROBIOL. 1986, 132(11), 3221-9

PROC. NATL. ACAD. SCI., vol. 83, July 1986, pages 4631-4635; A. NICOSIA et al.: "Cloning and sequencing of the pertussis toxin genes: Operon structure and gene duplication"

INFECTION AND IMMUNITY, vol. 42, no. 1, October 1983, pages 33-41, American Society for Microbiology; A.A. WEISS et al.: "Tn5-induced mutations affecting virulence factors of Bordetella pertussis"

INFECTION AND IMMUNITY, vol. 55, no. 10, October 1987, pages 2465-2470, American Society for Microbiology; W.J. BLACK et al.: "Construction and characterization of Bordetella pertussis toxin mutants"

**Description**

This invention relates to pertussis vaccines, particularly vaccines produced by genetic engineering processes.

Bordetella pertussis is a small gram-negative bacillus found only in humans. It is the etiologic agent of the childhood disease whooping cough, also known as pertussis.

In susceptible individuals, the disease may progress to a serious paroxysmal phase. Violent and spasmotic coughing occurs, with the patient be subject to secondary injury from the hypoxia, hypertension, and convulsions attendent with the coughing paroxisms. Secondary infections, encephalopathy, and death may occur.

The discrete molecular moiety that has been associated with these severe effects in the paroxysmal stage of the disease is pertussis toxin (Ptx). Ptx has been reported under a variety of names, including lymphocytosis promoting factor, histamine sensitizing factor, and islet-activating protein.

A specific biochemical reaction has been described for Ptx, which is believed to be the mode of action for the physiological effects of the toxin. Ptx transfers ADP-ribose from NAD to certain eukaryotic guanine-nucleotide-binding proteins, thereby disrupting their normal functions. A number of other biochemical affects have also been noted.

Pertussis toxin is an oligomeric structure which basically conforms to the A-B model of bacterial toxins. The sequence of the toxin is known. There are five peptide subunits, designated S1, S2, S3, S4, and S5, which are present in holotoxin in a 1:1:1:2:1 stoichiometry. S1, the "A" monomer, catalyzes the ADP-ribosylation reaction of the toxin. The B oligomer, composed of the other four subunits, is thought to be responsible for binding of the toxin to host cell membranes and perhaps for initiating entry of S1 into the cell.

Prior to the present invention, little was known of how manipulation of ptx genes would alter Ptx or its toxicity. Falcow and co-workers initially described the genetic locus which encodes pertussis toxin. This locus, ptx, was identified by a transposon insertion which enactivated expression of one of the pertussis toxin subunits, S3. Two other groups have recently cloned, and by sequence analysis identified, B. pertussis DNA which when translated corresponds to Ptx subunit amino acid sequences. The two cloned sequences are essentially identical and contain open reading frames for all five Ptx subunits arranged contiguously in an operon-like structure. Neither group has reported expression of the cloned sequences or genetic modification of the ptx locus. A transposon insertion mutant of Bordetella had been isolated which exhibited altered partitioning of ptx subunits between intracellular and extracellular fractions. At least one trans-acting locus, vir, was known to be necessary for toxin biosynthesis.

A crude by effective pertussis vaccine consisting of heat-killed organisms was developed in the 1930's and 1940's. Widespread use of this vaccine led to a dramatic decrease in the incidence of the disease. Recently, a resurgence of pertussis incidences has occurred, caused by decreased public acceptance of the vaccine. This decline in vaccine acceptance reflects a concern for the adverse affects associated with immunization using the heat-killed vaccine. Adverse affects range from minor irritability to convulsions, coma, and, on rare occasions, death. Children who recover from the severe reactions show varying degrees of permanent neurological defects, including mental retardation.

The vaccine in general use today is identical to that developed 50 years ago. Advances in the field of pertussis immunoprophylaxis have primarily been in quantitation of vaccine potency and toxicity. An acellular but still poorly defined vaccine, which includes Ptx, is currently undergoing trials in Japan. No results are yet available.

Accordingly, there remains a need for new pertussis vaccines capable of inducing immunity against pertussis but lacking the adverse affects of current pertussis vaccines.

Relevant Publications

The oligomeric structure of pertussis toxin was first described in Tamura et al. (1982) Biochemistry, 21:5516. The nucleotide sequence and genetic organization of the pertussis toxin gene is described in Locht et al. (2986) Science, 232:1258-1264. Cloning and sequencing of the pertussis toxin genes is discussed along with operon structure and gene duplication in Nicosia et al. (1986) Proc. Natl. Acad. Sci. USA, 83:4631-4635. A transposon insertion mutant of Bordetella is described in Weiss et al. (1983) Infect. Immun., 42:33, which also describes the transacting vir locus necessary for toxin biosynthesis. A recently developed mouse model for pertussis-vaccine-associated encephalophathy is described in Steinman et al. (1982) Nature, 299:738. Correlational studies between vaccine composition and potency suggesting that Ptx is a primary vaccine component are set forth in Pittman (1984) Ped. Infect. Dis., 3:467. A publication setting forth numerous articles relevant to pertussis vaccines is "Developments in Biological Standardization", Volume 61, Mann, Clark, and Hennessen, eds., S. Karger, Basel, 1985. The papers in this book are derived from the proceedings of the Fourth International Symposium on Pertussis held in Geneva, Switzerland, in 1984.

Mutations have been introduced into the Bordetella pertussis chromosome in the toxin gene which alter the toxicity of the toxin molecules produced by the organism while retaining immunogenicity. Two unmarked mutations, ptx3201 (with an insertion in the S1 subunit) and ptx058 (with the entire S1 subunit being deleted), are particularly important for vaccine purposes.

The nucleotide sequences utilized in the present invention are derived from genetic information isolated from Bordetella pertussis. The present invention includes modifications of the natural genes and polypeptide gene products, particularly those gene products that retain immunogenicity but lack the virulence of wild-type pertussis toxin. A vector useful for inserting mutations into the chromosome of B. pertussis is also provided.

This invention will be better understood by reference to the following detailed description when considered in combination with the drawings provided, wherein:

Figure 1 is a chromosomal restriction map of the ptx region and further shows various deletion mutations.

Figure 2 is a construction scheme for pRTP1. Arrowheads indicate restriction sites involves at each step.

Figure 3 is a physical map of pRTP1 showing positions of restriction enzyme cleavage sites. Positions marked Jnxn. indicate the points where a BamHI site was joined to a BglII site such that neither site was maintained.

Figure 4 is a schematic of pTOX13 and its relationship to the B. pertussis chromosome. pTOX13 is shown above and in register with a restriction map of the corresponding region of the chromosome. The sites of the introduction of the ptx5171 and ptx3201 alleles are shown. DNA sequences corresponding to the w.t. and ptx3201 alleles are shown in the upper left corner.

Figure 5 is a schematic diagram of the process of replacement of ptx5171 in the chromosome of BP370 with ptx3201 on pTOX13-ptx3201. Resistance phenotype of the strain at different stages is shown to the right.

The present invention has been reduced to practice in part by identifying and cloning the genetic locus encoding pertussis toxin. At least one toxin subunit has also been expressed in E. coli by genetic engineering techniques. Furthermore, in vitro mutagenesis and allelic exchange have been used to create B. pertussis strains deficient in production of the toxin while still being capable of stimulating an immunogenic response.

Specifically, Bordetella pertussis toxin genes have been expressed by ligational fusion of the pertussis toxin (Ptx) operon genes to the hybrid promoter tac. The Ptx operon genes were initially identified by fusion of the cloned ptx locus to a tac promoter vector. This fusion plasmid was designated pTOX1. Plasmid pTOX1 expresses at least one Ptx subunit in E. coli. Clones were initially detected with a rabbit antiserum in a colony blot radioimmunoassay. Specific production of Ptx subunit S4 was demonstrated utilizing an S4-specific monoclonal antibody in a radioimmune precipitation of labelled products from a pTOX1-charged in vitro transcription/translation system. The DNA sequence of the Ptx operon indicates that PTOX1 fused the tac promoter to a point about 200 base pairs 5' from the 3' end of the S1-subunit open reading frame. This work has demonstrated the utility of E. coli as a host for the large-scale production of pertussis toxin and has opened the way to production of Ptx (in natural or modified form) in other hosts.

As noted above, the expression of pertussis toxin in B. pertussis is normally vir dependent. By operably inserting an appropriate promoter upstream from a gene encoding a pertussis toxin subunit, or a number of genes encoding the various subunits of a complete pertussis toxin, it is possible to constituitively produce the subunit by culturing the modified strain and isolating the subunit from the culture medium. The pertussis toxins can be isolated by standard means for isolating proteins from bacterial culture media, which may include disrupted cells or may be limited to soluble products secreted into the culture medium. These methods include but are not limited to differential precipitation, gel filtration, and affinity chromatography, which may be based on use of a monoclonal antibody to a specific subunit, an antiserum prepared against entire pertussis toxin or the like.

It is also possible to combine an insertion of a tac promoter into a gene construction with a chromosomal deletion of a S1 pertussis toxin subunit gene. A strain possessing such a construction will express only the B oligomer subunits. Such B oligomer subunits can be used as an active vaccine to generate antibodies or may be used to generate antibodies in vitro for use in a passive vaccine.

The information available as a result of the present invention enables those skilled in the art to make intelligent selections about bacterial strains in which to produce pertussis toxin. Sequence analysis of the pertussis toxin operon has indicated a guanine plus cytosine content (65%) considerably higher than that of the traditional E. coli cloning host (approximately 50%). Accordingly, production of pertussis toxin in other species in genera can be facilitated by selecting for use as a host a species or genera having a high guanine plus cytosine content preferably at least 60%, more preferably at least 65%. The Bordetella and Pseudomonades genera are particularly suitable. Of the four Bordetella species (avium, bronchiseptica, parapertussis and pertussis), parapertussis is preferred as a host since it is not associated with an infectious disease.

The pertussis toxin from B. pertussis has been introduced into B. parapertussis as either a tandem merodiploid chromosal cointegrate or on a P-incompatibility-group episome. In both of these cases pertussis toxin has been expressed as determined by a colony blot radioimmunoassay.

Based on the genetic information now available, it is possible to introduce specific mutations into the pertussis toxin operon which lead to altered toxin biosynthesis phenotypes. Starting with a cloned pertussis toxin operon, in vitro mutagenesis and allelic exchange can be utilized to introduce defined genetic lesions into the B. pertussis chromosome.

A number of different types of mutations can be specifically introduced into the pertussis toxin operon. These include marked polar deletions, marked polar insertions, unmarked nonpolar codon insertions, and unmarked polar and nonpolar deletions. Those skilled in the art, using the information provided in this specification, can readily prepare variations of the following types of mutations, which are included here for purpose of illustration.

For example, marked polar deletions and insertions can be carried out utilizing a 1.2-kilobase-pair, polylinker-flanked aminoglycoside phosphotransferase (ABT II) gene as the marker. Such mutations can be constructed in a plasmid such as pTOX9 for allelic exchange return to B. pertussis.

Unmarked nonpolar codon insertions and unmarked polar and nonpolar deletions can likewise be constructed utilizing a shuttle vector, such as pRTP1, for allelic exchange return to B. pertussis.

Although a number of types of allelic exchange can be visualised, the following technique has proven satisfactory and is exemplory of operable techniques. A plasmid derivative containing the P-incompatibility-group origin of transfer, rlx, that is transmobilized by a suitable plasmid can utilized in the technique of conjugated mobilization. A suitable plasmid for transmobilization is pRKTV5. Exchange of marked mutations can be directly selected and scored, while exchange of unmarked mutations is a two step process.

The present invention has demonstrated the feasibility of selectively deleting genes from the B. pertussis chromosome and thus selectively deleting phenotypes from B. pertussis. The invention has demonstrated the pertussis toxin genes are not critical to in vitro B. pertussis viability and that strains deficient in the production of pertussis toxin can be routinely grown. Accordingly, mutants which produce only a limited number of the pertussis toxin subunits or an altered holotoxin may be used themselves as vaccines without the adverse affects associated with wild-type pertussis toxin.

The word "artificial" as used in describing an artificial nucleotide or peptide sequence is intended to exclude natural products, such as entire nucleotide sequences when present in Bordetella pertussis. 'Artificial' here indicates that some manipulation by human beings has taken place. Purification and isolation of a particular nucleotide or peptide sequence is a sufficient manipulation. The nucleotide sequence can be DNA, or RNA, and may be either identical to or complementary to a natural Bordetella pertussis sequence. Both single- and double-stranded DNA and RNA are encompassed by the present invention, with single-stranded RNA or DNA being useful as intermediate products.

Double-stranded nucleotide sequences are particularly useful for the production of proteins by introduction into appropriate vectors. Such proteins have use either as vaccines or as components of vaccines. Particularly useful are sequences that code for a complete mature subunit of pertussis toxin, including any of the S1 - S5 subunits. It is also possible for the sequence to include within it several sequences that code for individual subunits. One preferred sequence encodes an entire B subunit of pertussis toxin. Other preferred sequences code for at least one but no more than three of the four subunits that make up a wild-type B subunit of pertussis toxin.

This invention has been further reduced to practice by creating mutants of B. pertussis with altered pertussis toxin phenotypes. The chromosomal locus ptx, previously identified by transposon mutagenesis, was verified to be the Ptx coding region. Cloned ptx DNA placed under control of the tac promoter expressed Ptx antigens in E. coli and in an E. coli, in vitro transcription-translation system. The ptx locus restriction map was found to be identical to the known cloned sequences of B. pertussis DNA. B. pertussis ptx mutants were created by in vitro mutagenesis of cloned Ptx coding regions, followed by allelic exchange to introduce the mutations into the B. pertussis chromosome. The introduction and position of the mutations was confirmed by Southern transfer. These Ptx mutants enable analysis of the toxin's contribution to pertussis pathogenesis and immunity.

The pertussis toxin mutants demonstrated two major features of pertussion toxin biogenesis that are useful in designing vaccines. The first is that biogenesis of toxin subunits S2 and S4 requires an intact and uninterrupted S1 open reading frame. This appears to indicate that the first three genes of the operon form a single transcriptional unit, i.e., they are polycistronic. While the prior art had indicated that two promoters are present, one preceding the S1 open reading frame and the other preceding the S4 open reading frame, data from the present studies indicates that only the promoter preceding the S1 open reading frame is used. Alternatively, it is possible that S2 or S4 are independently transcribed but require the S1 message or product as a transcriptional activator. Another possibility is that S2 and S4 are unstable in the absence of S1. Nonetheless, it is clear that for biogenesis of subunits S2 and S4, an intact S1 gene is required.

Another major feature of pertussis toxin biosynthesis disclosed by these mutations is that interruption of the 3' subunit open reading frames leads to either incomplete synthesis or product instability of subunits encoded 5' to the interruption. More specifically, mutation of the S5 subunit open reading frame leads to production of a truncated S1 subunit; mutation of the S4 open reading frame leads to a lack of detectable S2 subunit. This relationship of the subunits in biogenesis directly reflects the physical associations of the subunits. Subunits S2 and S4, and subunits S3 and S4, assemble into relatively stable heterodimers. The two heterodimers, S2/S4 and S3/S4, in the presence of S5 will then combine with S5 to form a heteropentamer, S2/S4-S5-S3/S4. This heteropentamer is a stable structure and is believed to mediate binding of the toxin to host tissue. It has been used in vitro to block activity of the holotoxin. The heteropentamer will then stably associate with the S1 subunit. In the absence of S5, the S1 subunit will not associate with any of the other subunits. Also, in the absence of subunit S4, S2 will not associate with any of the other subunits. These physical relationships among the toxin subunits are directly reflected in the stable biogenesis of the subunits. The truncation of the S1 (A) subunit of pertussis toxin in the absence of the S5 subunit and the other instabilities described herein allow rational design of vaccines by producing subunits that stably interact.

For example, if a vaccine containing some but not all of the S1-S5 subunits is desired, it appears that a S5 subunit must be present if a S1 subunit is to be included. Likewise, a S4 subunit should be present if a S2 subunit is desired. Accordingly, preferred combinations of subunits include S1/S5, S5 alone, S2/S4, S4 alone, S1/S4/S5, S2/S4/S5, and S1/S2/S4/S5. Also preferred are combinations of any of these subunit groupings (except for the last subunit grouping S1/S2/S4/S5) with the S3 subunit. It should be noted that these groupings are not intended to be stoichiometric. However, stoichiometric ratios approaching the natural ratios of 1:1:1:2:1 for the S1, S2, S3, S4 and S5 subunits, respectively, are preferred.

A vector specifically designed to facilitate the return of cloned and/or altered sequences to replace the corresponding sequence in the B. pertussis chromosome was constructed. This plasmid is designated pRTP1 (Return to Pertussis). Using this vector one can select for the integration of this vector via homologous recombinations and subsequently for its loss through the same mechanism. Plasmid pRTP1 thus provides the ability to easily replace a chromosomal allele with a cloned allele.

pRTP1 contains the following elements: oriT, the origin for conjugative transfer from the broad host range plasmid RK2; a vegetative origin of replication derived from ColE1; a gene encoding resistance to ampicillin; the gene for E. coli ribosomal protein S12 which renders streptomycin resistant strains streptomycin sensitive; and the cos site of bacteriophage lambda.

oriT allows pRTP1 containing cloned B. pertussis sequences to be complemented for the ability to transfer via conjugation from E. coli into recipient B. pertussis strains. Once transferred this DNA is unable to be maintained via the ColE1 origin of replication and thus if selection for ampicillin resistance is imposed only those bacteria in which the incoming plasmid has been integrated into the chromosome will survive. This integration will normally occur via homologous recombination within the cloned B. pertussis sequences. If the B. pertussis recipient contains the streptomycin resistance allele of the gene for ribosomal protein S12, imposing selection for resistance to streptomycin now will select for those bacteria in which the vector has been lost due to a second crossover within the cloned sequences. If the mutation being introduced has a selectable marker, maintaining selection for that marker during the streptomycin selection will ensure that all streptomycin resistant survivors have incorporated the mutation. By virtue of the cos site pRTP1 can also be used as a cosmid cloning vector for the construction of gene banks. Thus a single vector may be used for the initial cloning of large sequences of the B. pertussis chromosome, the analysis and alteration of these sequences in E. coli, and the return of these altered sequences to replace their chromosomal counterparts.

Surprisingly the E. coli gene for ribosomal protein S12 is expressed efficiently in B. pertussis, and the S12 protein made is apparently capable of being assembled into B. pertussis ribosomes. This has proved extremely useful and suggests that pRTP1 may also function properly in other bacterial species where ColE1-based plasmids fail to replicate (Mejean et al., Gene (1981) 15:289-293; Niaudet et al.,Gene (1982) 19:277-284; Shimkets et al., Proc. Natl. Acad. Sci. USA (1983) 80:1046-1410; Williams and Szalay, Gene - (1983) 24:37-51). In E. coli and in Salmonella species mutations in the polA gene render ColE1-derivatives incapable of replication. In these species streptomycin resistant derivatives are also readily available, and thus pRTP1 may be used in them for allele replacement as well as for determination of the essentiality of a given gene.

A number of workers have described the replacement of chromosomal DNA sequences with corresponding sequences cloned into plasmid and bacterophage vectors (Lee and Saier, J. Bacteriol. (1983) 153:685-692; Williams and Szalay, Gene (1983) 24:37-51; O'Connor and Zusman, J. Bacteriol (1983) 155:317-329; Gutterson and Koshland, Proc. Natl. Acad. Sci. USA (1983) 80:4894-4898; Joyce and Gridley, J. Bacteriol. (1984) 158:636-643; Pozzi and Guild, J. Bacteriol. (1985) 161:909-912. These schemes have generally depended on the non-replication of those vectors within the recipient bacterium together with a marker to allow selection for integration via homologous recombination. In many cases this selection results in the duplication of the cloned sequences in the chromosome flanking the cloning vector. To complete the process of allelic exchange in these cases, a second recombination event within the cloned sequences is necessary to remove the vector and leave a single allele. These progeny can be obtained by screening candidates for loss of the vector. The method of Joyce and Grindley (1984), supra, provides an important advantage in that loss of the phage vector can be selected for by resistance to killing after thermal induction of the prophage. The system described here is analgous in that a negative selection on pRTP1 is included. For the negative selection advantage has been taken of the fact that resistance to high levels of streptomycin, due to a mutant allele of the rpsL gene, is recessive to the sensitive allele. The necessity of first introducing a str$^R$ allele into the B. pertussis strain under study is not a major limitation as mutations of the proper type are easily obtained by selection for spontaneous resistance to high levels of streptomycin.

It should be noted that one possible difficulty with the use of pRTP1 may arise if the integrative recombination occurs within the S12 gene of B. pertussis. This would also give rise to ampicillin-resistant, streptomycin-sensitive derivatives. However, if either the chromosomal or the cloned allele is marked with an easily screenable marker, these can be easily distinguished in that no exchange of the markers will be possible. For example, in the case presented here of the introduction of an unmarked ptx mutation, we could be assured that integration was via homology in ptx as streptomycin-resistant, kanamycin-sensitive derivatives could be obtained.

The invention will now be described by reference to examples of certain embodiments which are set forth for purposes of illustration and are not to be considered limiting unless otherwise specified.

EXAMPLE 1: Mutants

Materials and Methods

Strains and Media.

Bacterial strains and plasmids used are identified in Table 1.

## TABLE 1

| Strains | Relevant Characteristics |
|---|---|
| **B. pertussis** | |
| BP370 | $ptx^+$, $rif^r$, $str^r$, derived through multiple passages from Tohama III (Reference). |
| TOX3201 | $ptx$3201 |
| TOX3300's | contain $ptx$3300's |
| TOX5100's | contain $ptx$5100's |
| TOX5301 | $ptx$5301 |
| **E. coli** | |
| HB101 | $rif^s$ |
| **Plasmids** | |
| pHC79 | cosmid vector |
| p11.11 | $ptx$ region cloned into pHC79 |
| pRI133 | ptac12 with polylinker flanked APT II gene inserted at $EcoRI$ site |
| pRTP1 | ColEI $oriV$, $rpsL$ ($str^s$), $amp^r$ |
| pEYDG1 | contains P inc $rlx$ |
| pKAN | contains polylinker flanked APT II gene |
| pRKTV5 | P inc Tra functions |
| pTOX1 | $ptx$ cloned into pRI133 |
| pTOX9 | cloned $ptx$, ColEI $oriV$, P inc $rlx$ |
| pTOX13 | cloned $ptx$ in pRTP1 |

Eschericia coli were cultured on L agar, and B. pertussis were grown on Bordet Gengou agar with 15% sheep blood, both at 37°C. B. pertussis were cultured in loosely stoppered jars to maintain high humidity; otherwise atmosphere was ambient. Antibiotics were included when appropriate at the following concentrations: ampicillian, 100 $\mu$g/ml for E. coli and 40 $\mu$g/ml for B. pertussis; kanamycin, 40 $\mu$g/ml; rifampicin, 50 $\mu$g/ml; streptomycin, 300 $\mu$g/ml.

Molecular biology.

Standard cloning techniques were utilized. Generation of random DNA fragments was by sonication. Incremental deletions were products of Bal 31 exonuclease digestion. Partial digestion to cut plasmids at one of a multiply represented restriction site sequence was accomplished in the presence of ethidium bromide. Linkers were purchased from Pharmacia. Restriction endonuclease and other DNA-modifying enzymes were from either New England Biolabs or BRL. Plasmid pKAN was constructed for production of the polylinker flanked APT II gene and was an insertion of the polylinker flanked gene into the EcoRI site of pBR322. Southern transfer hybridization was at 68°C without formamide; the final wash was in 0.1 x SSC, 0.1% SOS at 68°C.

Standard techniques were utilized for colony blot radioimmune assay, radioimmune precipitation, polyacrilamide gel electrophoresis, and Western transfer analysis. In radioimmune precipitations we utilized Pharmacia Immunobeads. $^{35}$S methionine was purchased from Amersham. Polyacrylamide gels were 15%. Western probe buffer was BLOTTO. B. pertussis strains grown on Bordet Gengou agar were prepared for electrophoresis by suspension in water followed by heating in solubilization buffer at 100°C. Rabbit antiserum to pertussis toxin and monoclonal antibodies specific for pertussis toxin subunits were obtained by established techniques. Antiserum probed transfers were labeled with Amersham $^{125}$I Protein A. Monoclonal antibody probed transfers were labeled with Amersham radioiodinated Goat anti-Mouse Ig. In vitro transcription-translation reagents from E. coli HB101 were utilized.

### Genetics.

Conjugative mobilization of plasmids into B. pertussis was carried out as follows. Eighteen-hour E. coli and 60-hour B. pertussis plate cultures were swabbed together onto Bordet Gengou agar plates containing 10.0 mM MgCl$_2$. Plates were incubated at 37°C for 3.5 hours in loosely stoppered jars followed by reswabbing onto selective media. Rifampicin was typically used for counter-selection against E. coli. The two step allelic exchange procedure to introduce unmarked mutations utilizing pRTP1 is described in Example 2.

The CHO cell clumping assay for pertussis toxin activity was employed.

### Results

### Expression of Ptx in E. coli.

Plasmid pTOX1 was recovered from a cloning of randomly generated fragments of p11.11 into the PvuII site of pRI133. This placed B. pertussis DNA adjacent to 3' of the strong hybrid tac promoter. E. coli strains HB101 and SE5000 harboring pTOX1, but not those harboring pRI133, bound Ptx antisera by colony blot radioimmunoassay (data not shown). More specifically, that pTOX1 encoded Ptx subunit S4 was demonstrated by radioimmune precipitation. The plasmids were used to charge in vitro transcription-translation reactions, the products of which were labelled with $^{35}$S methionine and subsequently precipitated with a monoclonal antibody specific for Ptx subunit S4. Polyacrylamide gel electrophoresis indicated that labelled, precipitated material encoded by pTOX1 migrated with the apparent molecular weight for S4.

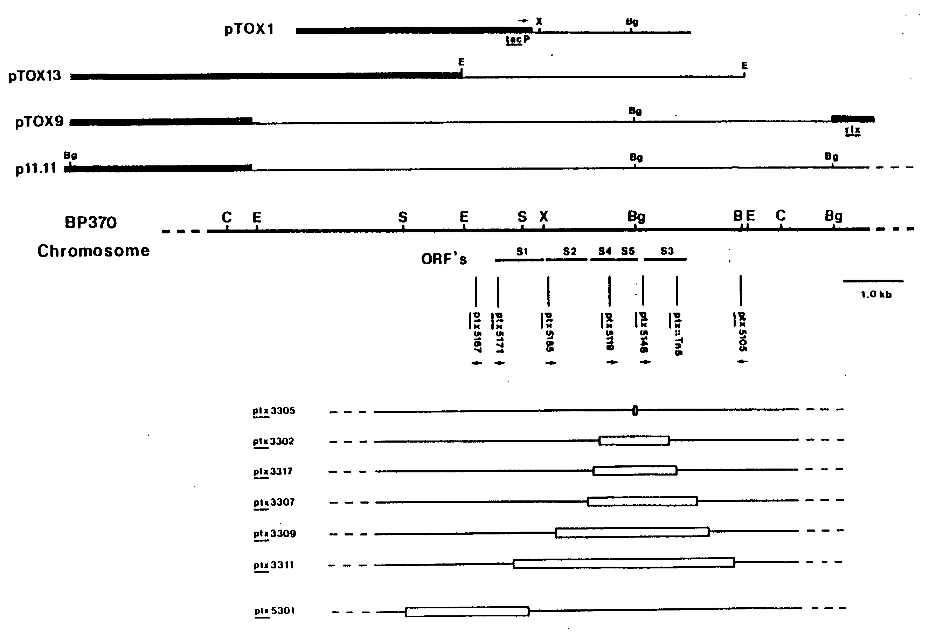

Restriction mapping of the chromosomal region cloned in pTOX1 indicated that it included the ptx::Tn5 insertion previously reported (Fig. 1). Further, the map coincides with those reported by other groups (see Relevant Publications section).

### Construction of ptx mutations.

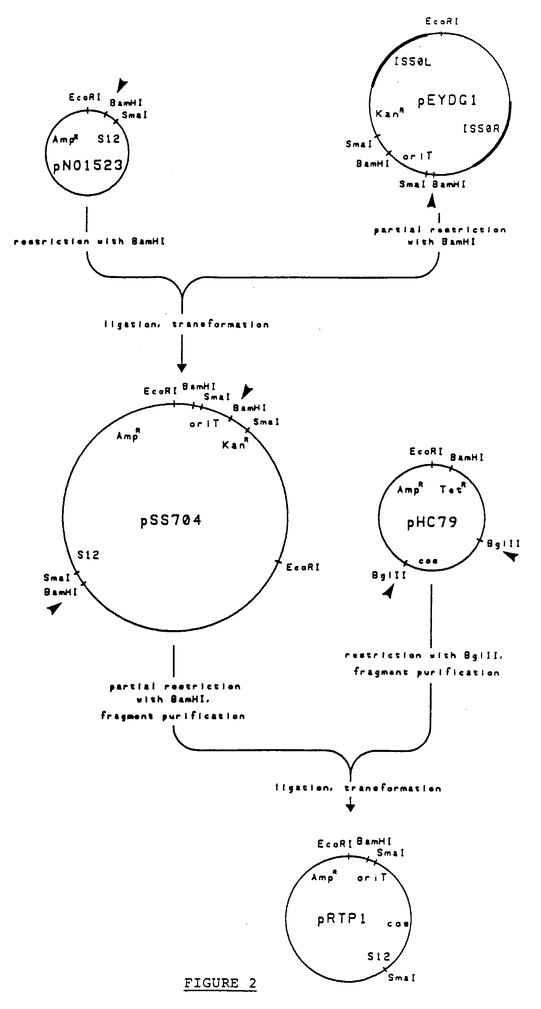

Shuttle vectors utilized for construction of mutations and subsequent introduction into B. pertussis are depicted in Figure 2. Plasmid pTOX9 was constructed to introduce marked ptx mutations into the B. pertussis chromosome, and included two adjacent BglII restriction fragments of p11.11, providing all of the ptx region as well as the Col EI ori V and bla gene from pHC79. The P incompatibility group origin of transfer, rlx, was introduced into the BglII site found at the junction of B. pertussis and vector DNA, as a 0.7 kb BamHI fragment from pEYDG1. This insertion destroyed this BglII site leaving only one such site in pTOX9. Unmarked mutations were delivered into B. pertussis utilizing pTOX13. This plasmid contained the 4.7 EcoRI restriction fragment ecompassing ptx, cloned into the vector pRTP1.

For each mutation, the mutation itself, the mutation-bearing plasmid, and the resultant B. pertussis mutant were given the same ptx, pTOX, or TOX number. The incremental deletions of the ptx3300 series were created in pTOX9, and were centered about the single BglII site. All deletions were marked by ligational insertion of the 1.5 kb APT II gene from pKAN into the deletion site. The deletion of ptx5301, also created in pTOX9, is a simple removal of the 1.9 kb SalI fragment followed by replacement with the APT II gene. The insertion mutations of the ptx5100 series were created by insertion of the APT II gene into various of the Sau 3A sites scattered about pTOX9. Figure 1 indicates the map positions and extent of the insertions and deletions with respect to the Ptx subunit coding regions previously delineated by sequencing.

An unmarked mutation was created in the S1 open reading frame. The mutation ptx3201 was generated by limited SalI digestion to open pTOX13 at the SalI site within the S1 ORF, followed by end-filling and blunt ligation of an eight base pair linker CGGATCCG into the breach. This operation introduced a sum total twelve base pairs, maintaining reading frame integrity, and introducing a BamHI site to monitor the construction. Note also that this construction duplicated the SalI site and that introduction of two or more linkers would introduce a novel SstII site. This latter feature was useful in assuring that only a single linker was added in the TOX3201 construction. The twelve base pair addition translated into a four amino acid insertion, val-asp-gly-ser, into the S1 sequence.

Detailed Description of Ptx Clones and Genetic Constructions as Shown in Figure 1.

BP370 Chromosome denotes a chromosomal restriction map of the ptx region. Restriction sites are C, ClaI; E, EcoRI; S, SalI; X, XbaI; Bg, BglII; B, BamHI. Plasmids pTOX1, pTOX13, pTOX9, and p11.11, described in the text, contain cloned ptx DNA. B. pertussis derived DNA is denoted by a thin line; vector DNA is denoted by a thick line. Vector sequences of pTOX13 and p11.11 have been truncated to facilitate inclusion in the diagram. The position and direction of the tac promotor (tacP) in pTOX1 is indicated. Positions of the ptx5100 series insertion mutations and the ptx::Tn5 insertion of BP357 are indicated. Arrows below the ptx5100 series insertions denote the transcriptional direction of the inserted APT II gene. The open rectangular boxes of the ptx3300 series deletions and deletion ptx5301 indicate extent of deletions.

Positions of insertions from EcoRI site immediately 5' of S1 ORF.

| ptx5167 | 0.19 kb |
|---------|---------|
| ptx5171 | 0.55 kb |
| ptx5185 | 1.4 kb |
| ptx5119 | 2.4 kb |
| ptx5148 | 2.9 kb |
| ptx5105 | 4.5 kb |

Extent of deletions measured from Eco RI site immediately 5' of S1 ORF.

| | Left end | Right end |
|---------|----------|-----------|
| ptx3305 | 2.8 kb | 2.8 kb |
| ptx3302 | 2.2 kb | 3.3 kb |
| ptx3317 | 2.1 kb | 3.5 kb |
| ptx3307 | 2.0 kb | 3.8 kb |
| ptx3309 | 1.5 kb | 4.1 kb |
| ptx3311 | 0.7 kb | 4.4 kb |
| ptx5301 | -1.0 kb | 0.93 kb |

Creation of B. pertussis ptx mutants.

Mutations were introduced into B. pertussis by conjugative mobilization and allelic exchange. Conjugative mobilization was accomplished by triparental matings between HP101 (pRK2013), HB101 (pTOX plasmids), and BP370. The pTOX plasmids, with ColEI origins or replication, when introduced into B. pertussis were unable to replicate, and selected markers were maintained only via plasmid cointegration into the chromosome. All cointegrates examined formed via homologous recombination between the cloned and chromosomal ptx loci. In conjugations with plasmids pTOX5301, and of the pTOX3300, and pTOX5100 series, selection for the kanamycin resistance marker with subsequent scoring for ampicillin sensitivity yielded B. pertussis exconjugants which had undergone a double crossover. These mutants had lost the wild-type ptx locus and gaind the mutated version.

The mutation ptx3201 was introduced into the ptx locus by conjugative mobilization of pTOX3201 into B. pertussis strain TOX5171. The initial selection for kanamycin and ampicillin resistance yielded exconjugates with a cointegration of pTOX3201 into the TOX5171 chromosome formed via homologous recombination between plasmid and chromosomal ptx loci. Subsequent selection for streptomycin resistance, followed by scoring for kanamycin and ampicillin sensitivity, yielded a mutant which had lost the plasmid sequences and the ptx5171 mutation, and had gained the ptx3201 mutation. This mutant was designated TOX3201.

Southern transfer was used to confirm the introduction of mutations into the exconjugants' chromosomes. The APT II gene marker contains a single ClaI site splitting it into 1.0 and 0.5 kb fragments The ptx locus is located on a 10.0 kb chromosomal ClaI fragment. Chromosomal DNA from B. pertussis ptx mutants was digested with ClaI and probed with either the 10.0 kb ClaI fragment from the ptx region or with the 1.5 kb APT II gene. The combined sizes of the hybridizing bands in the TOX3300 mutants indicated incremental chromosomal deletions and a 1.5 kb APT II gene insertion. The combined size of the hybridizing bands from TOX5301 likewise indicated a deletion and APT II gene insertion. The varying individual sizes, but constant sum total size of the hybridizing bands of the TOX5100 mutants indicated varying chromosomal insertions. TOX3201, which contained no APT II marker was analyzed by digesting chromosomal DNA with BamHI and probing with the 4.6 kb EcoRI/BamHI ptx-containing fragment. Wild-type ptx lies on a 15.0 kb BamHI fragment. Introduction of the ptx3201 BamHI linker mutation appropriately divided that into 3.6 and 10.4 kb fragments.

B. pertussis Ptx mutant phenotypes.

B. pertussis ptx mutants were scored for production of Ptx subunits with monoclonal antibodies specific for individual toxin subunits. The mutants were scored by Western transfer. Each Western included control lanes containing purified Ptx probed with antiserum to Ptx holotoxin and purified Ptx probed with the monoclonal antibody (McAb). B. pertussis ptx strains could be divided into three classes with respect to their S1 subunit phenotypes. Certain strains manifested both native size (ca 28,000 kilodaltons) and reduced size (ca 20,000 kilodaltons) S1-McAb-binding bands. These included BP370, TOX5167, TOX5148, and TOX5105. Certain strains manifested only the reduced size S1 McAb binding band. These included TOX5119, TOX3305, TOX3302, and TOX3316. Finally, two strains, TOX5171 and TOX3311, manifested no S1 McAb binding bands. It is important to note that no reduced size band was present in the purified toxin lane.

B. pertussis strains were also scored with S2 and S4 specific monoclonal antibodies. S2 and S4 phenotypes parallelled each other. Strains BP370, TOX5167, TOX5148, TOX5105, TOX3305, and TOX3316 all manifested both S2 and S4 subunits of appropriate apparent molecular weights. Strains TOX5171, TOX5119, TOX3302, and TOX3311 manifested no S2 or S4 bands.

B. pertussis ptx mutant TOX3201 was also scored for Ptx subunit phenotype. TOX3201 produced appropriate sized antigenic material corresponding to subunits S1, S2, and S3. Specific emphasis was placed on comparing the electrophoretic mobility of S1 from TOX3201 and S1 from wild type strain BP370. The ptx3201 insertion of val-asp-gly-ser into the S1 amino acid sequence should add 429 daltons molecular weight to the S1 subunit. Western analysis indicated that the S1 from TOX3201 was in fact about 400 daltons larger in apparent molecular weight than the S1 from BP370. Note also that a reduced size band of ca 20,000 kilodaltons is also present in the TOX3201 lane, but is also about 400 daltons larger than the analogous band in the BP370 lane.

Supplemental Description of ptx Mutant Phenotypes

These descriptions are derived from colony blot and Western transfer analyses of the B. pertussis ptx mutants.

A Ptx subunit biosynthesis phenotype may be indicated as either + or - (e.g., S1$^+$ of S1$^-$). Mutants which make either the entire toxin or no toxin are, respectively, Ptx$^+$ or Ptx$^-$. Probable phenotypes are in parentheses. Where no indication is given, the particular subunit phenotype is not known.

| Mutant | Phenotype | | | |
|--------|-----------|---|---|---|
| TOX5167 | Ptx$^+$ | | | |
| TOX5171 | S1$^-$, S4$^-$ | (S2$^-$, | S3$^+$) | |
| TOX5185 | S1$^-$, S4$^-$ | (S2$^-$, | S3$^+$) | |
| TOX5119 | S1$^-$, S4$^-$ | (S2$^-$, | S3$^+$) | |
| TOX5148 | S1$^+$, S4$^+$ | (S2$^+$, | S3$^-$) | |
| TOX5105 | S1$^+$, S4$^+$ | (S2$^+$, | S3$^-$) | |
| TOX3305 | S1$^-$, S4$^+$ | (S2$^+$, | S3$^-$) | |
| TOX3302 | S1$^-$, S4$^-$ | ? | ? | |
| TOX3317 | S1$^-$, S4$^-$ | ? | ? | |
| TOX3307 | S1$^-$, S4$^-$ | ? | ? | |
| TOX3309 | S1$^-$, S4$^-$ | ? | ? | |
| TOX3311 | Ptx$^-$ | | | |
| TOX5301 | S1$^-$ | ? | ? | |

Supplemental Description of TOX5301 pTOX12

TOX5301 pTOX12 is a strain of Bordetella pertussis constructed such that the pertussis toxin (Ptx) S1 subunit is not expressed and the B oligomer subunits are expressed under control of a tac promoter. TOX5301 pTOX12 is based upon two previous constructions: B. pertussis strain TOX5301 and pTOX12.

Plasmid pTOX12 was constructed as a vector to shuttle tac directed Ptx genes into B. pertussis. pTOX12 was derived from pTOX1 by first excising the 1.5 kb polylinker flanket APT II gene from pTOX1 as an BamHI fragment and inserting in its place the 0.7 kb BamHI containing fragment from pEYDG1 containing the P incompatibility group rlx locus. This yielded a ColEI ori V, amp$^r$, rlx containing plasmid with a tac promoter fused to the Ptx operon at a point ca 200 bp 5' from the XbaI site.

This plasmid was mobilized into TOX5301 from HB101, in a triparental mating utilizing HB101 (pRKTV5). Counerselection against HB101 was provided by the kan$^r$ marker in ptx5301. Selection for the amp$^r$ marker of pTOX12 yielded cointegrates of pTOX12 into the TOX5301 chromosome since ColE1 ori V will not replicate into B. pertussis. The structure of the cointegrate has been confirmed by Southern transfer.

These mutants also indicate several salient features of Ptx operon regulation and biosynthesis. The first and foremost is the polycistronic nature of the Ptx operon. The ptx5171 mutation, which lies near the 5' end of the S1 ORF, is polar for S1, S2, and S4 biosynthesis. Both reports of the operon sequence identify a primary promoter-like structure 5' of the S1 ORF. Keith and coworkers have further postulated the presence of a promoter preceeding the S4 ORF. The ptx5171 mutation would seem, however, to indicate that S4, as well as S2, uses only a promoter preceeding the S1 ORF. There is as yet no indication of the cistronic nature of the 3' end of the operon. The mutations ptx5167 and ptx5105 lie outside of the region encompassing the subunit ORF's, and thus serve as controls for APT II gene chromosomal insertions.

A second salient feature indicated by these mutations relates to the linked biosynthesis or biostability of toxin subunits. Certain subunits seem either incompletely synthesized or unstable in the presence of mutations in other 3' subunit ORFs. Mutations ptx3305 and ptx3316 lie well 3' of the S1 ORF, yet strains harboring these mutations manifested only a truncated form of subunit S1 at a concentration much reduced from wild type. A closer examination reveals that ptx3305 and ptx3316 lie in the S5 ORF. In contrast, the B. pertussis strain with the mutation ptx5148, which lies immediately 3' of the S5 ORF, produced wild type S1. Thus some aspect of the S5 sequence or subunit which is interfered with by mutations ptx3305 and ptx3316 is critical to S1 production or stability.

A second such linkage is also to be found with respect to S4 and S2. Mutations ptx5119 and ptx3302 lie 3' of the S2 ORF, yet strains harboring these mutations manifested no discernable S2. Mutations ptx5119 and ptx3302 lie in fact in the S4 ORF (and 3'). Again, in contrast, the B. pertussis strain with the mutation ptx3305, which lies 3' of the S4 ORF (in the S5 ORF), produced wild type S2. Thus some aspect of the S4 sequence or subunit, which is interfered with by mutations ptx5119 and ptx3302, is critical to S2 production or stability.

EXAMPLE 2: Pertussis Vector

Materials and Methods

a) Bacterial strains, plasmids, and media.

TABLE 2

Bacterial Strains and Plasmids

| Name | Genetic Markers |
|---|---|
| E. coli: | |
| HB101 | F⁻, hsdS20, recA13, ara-14, proA2, lacY1, galK2, rspL20, xyl-5, mtl-1, supE44 |
| LE392 | F⁻, hsdR514, supE44, supF58, lacY1, galK2, galT22, metB1, trpR55 |
| B. pertussis: | |
| BP369 | Rif^R, Str^R, vir⁻ |
| BP370 | Rif^R, Str^R, vir⁺ |
| BP370-ptx5171 | Rif^R, Str^R, Kan^R, vir⁺, ptx5171 |
| BP370-ptx3021 | Rif^R, Str^R, vir⁺, ptx3021 |
| Plasmids: | |
| pEYDG1 | Kan^R, oriT |
| pHC79 | Amp^R, Tet^R, cos |
| pNO1523 | Amp^R, rpsL |
| pRK290 | Tet^R, oriT, Tra⁺ |
| pRK2013 | Kan^R, oriT, Tra⁺ |
| pRKTV5 | Str/Spc^R, Tmp^R, Tra⁺ |
| pRTP1 | Amp^R, rpsL, oriT, cos |
| pSS617 | Amp^R, Tet^R, rpsL, oriT, IncP |
| pTOX13 | Amp^R, rpsL, oriT, cos, ptx |
| pTOX13-ptx3201 | Amp^R, rpsL, oriT, cos, ptx201 |

The strains and plasmids listed in this table are prepared as described in this application or in the following references: Maniatis et al., Molecular Cloning: A Laboratory Manual, Cold Spring Harbor Laboratory, Cold Spring Harbor, NY (1982); Weiss and Falkow, Infect. Immun. (1984) 43:263-269; Yakobson and Guiney, J. Bacteriol. (1984) 160:451-453; Hohn and Collins, Gene (1980) 11:291-298; Dean, Gene (1981) 15:99-102; Ditta et al., Proc. Natl. Acad. Sci. USA (1980) 77:7347-7351; Figurski and Helinski, Proc. Natl. Acad. Sci. USA (1979) 76:1648-1652; and Weiss and Falkow, Infect. Immun. (1983) 42:33-41.

Bacterial strains and plasmids used in this study are presented in Table 2. E. coli strains were maintained on L-agar (Miller, J.H.: Experiments in Molecular Genetics, Cold Spring Harbor Laboratory, Cold Spring Harbor, NY, 1972). Antibiotic concentrations for E. coli strains were: 100 mg/lit. for ampicillin, 40 mg/lit. for kanamycin, and 25 mg/lit. for tetracycline. B. pertussis strains were maintained on Bordet-Gengou agar (Difco) supplemented with 15% sterile defribinated sheep blood (Microbiological Media, Concord, CA). Unless otherwise noted, antibiotic concentrations for B. pertussis strains were: 20 mg/lit. for ampicillin, 40 mg/lit. for kanamycin, 50 mg/lit. for rifampicin, 25 mg/lit. for tetracycline, and 300 mg/lit. for streptomycin. All antibiotics were purchased from Sigma.

b) Enzymes and cloning techniques.

Standard procedures were applied using enzymes purchased from Bethesda Research Laboratories and New England Biolabs. BamHI linkers were purchased from Pharmacia Fine Chemicals. pTOX13-ptx3021 was constructed from pTOX13 by partial digestion with SalI in ethidium bromide, treatment with DNA-polymerase I Klenow fragment to create flush ends, and ligation with the BamHI linker 5'-CGGATCCG-3'.

c) Bacterial conjugations.

Biparental and triparental matings were performed by swabbing approximately $10^9$-$10^{10}$ bacteria of fresh plate cultures of each strain onto a Bordet-Gengou agar plate with no antibiotics. After three hours of incubation at 37°C in screw-top jars, bacteria were swabbed onto Bordet-Gengou agar containing the appropriate antibiotics for selection of exconjugants, and incubated in the same fashion. B. pertussis cultures used were three or four days old, E. coli cultures had grown overnight. Only dacron swabs were used for manipulation of B. pertussis.

d) Southern hybridizations.

Chromosomal DNA was prepared from B. pertussis strains by the method of J. Marmur, J. Mol. Biol. - (1961) 3:208-216. This DNA was restricted and subjected to electrophoresis and transfer to nitrocellulose membranes by the method of E. Southern, J. Mol. Biol. (1975) 98:503-517. Radioactivity labelled DNA probes were obtained using a nick-translation kit from Bethesda Research Laboratories.

Results and Discussion

a) Testing the Str$^S$ phenomenon in B. pertussis.

The positive selection cloning vector pNO1523 contains the gene for ribosomal protein S12 E. coli. To establish that this gene would be expressed in B. pertussis and would confer the streptomycin sensitive phenotype upon streptomycin resistant derivatives of B. pertussis, a fusion between pNO1523 and pRK290 was created by ligating pNO1523 cleaved at the single BamHI site with pRK290 cleaved at the BglII site. The recipient strain for transformation was LE392 [pRK2013]. The fusion plasmid obtained, pSS617, was transferred to the B. pertussis strains BP369 and BP370 (Table 2). These B. pertussis strains were rifampicin resistant as well as streptomycin resistant. Selection in the mating was for transfer of the tetracycline resistance of pRK290 and counterselection was by rifampicin resistance. As a control pRK290 was also mated onto these strains. Tetracyclin and rifampicin resistant exconjugants were obtained in all matings and were then tested for growth on medium containing other antibiotics. As shown in Table 3, the exconjugants containing pSS617 were ampicillin resistant as well as tetracycline resistant and failed to grow on media containing both tetracycline and streptomycin.

14

## TABLE 3

### Growth of Plasmid-containing B. pertussis
### Strains on Antibiotic Media

Growth on:

| Strain | BG-Tet | BG-Amp | BG-Tet,Str |
|---|---|---|---|
| BP369 [pRK290] | + | − | + |
| BP369 [pSS617] | + | + | − |
| PB370 [pRK290] | + | − | + |
| BP370 [pSS617] | + | + | − |

Strains were streaked onto Bordet-Gengou agar containing the indicated antibiotics. Antibiotic concentrations for this experiment were: tetracycline, 25 mg/lit; ampicillin, 50 mg/lit; streptomycin 50 mg/lit.

The exconjugants containing pRK290 were ampicillin sensitive, as they did not contain the beta-lactamase gene of pNO1523, but were streptomycin resistant, indicating that the streptomycin resistance allele was intact in BP369 and BP370. Thus, the S12 gene of pNO1523 containing the Str$^S$ allele is capable of being expressed in, and will confer a streptomycin sensitive phenotype upon streptomycin resistant B. pertussis strains.

b. Construction and physical mapping of pRTP1.

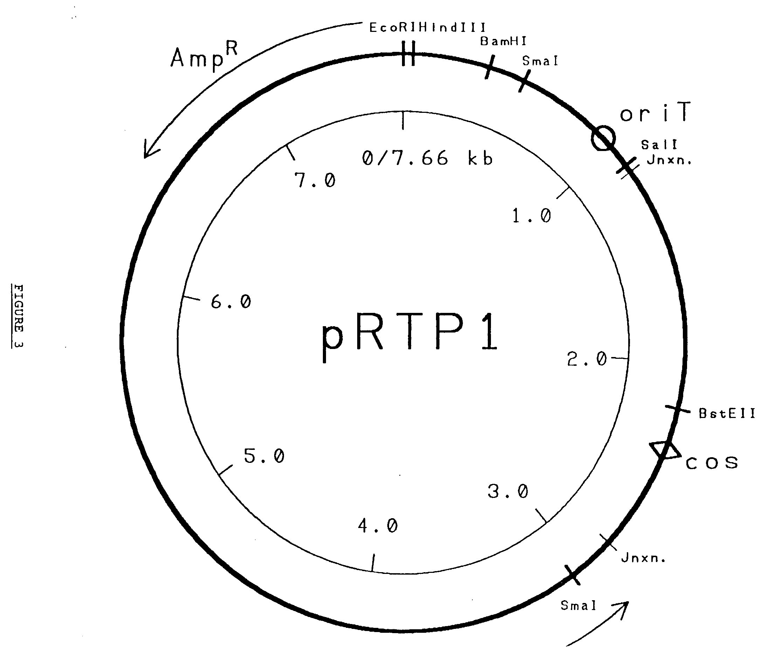

The construction of pRTP1 is diagrammed in Figure 2. In the first step a cointegrate was constructed between pNO1523 and pEYDG1 which contains oriT on a 769 bp BamHI fragment by ligating BamHI-cleaved pNO1523 with partial BamHI digestion products of pEYDG1. In the second step this fusion plasmid was again partially cleaved with BamHI and a fragment corresponding to pNO1523 plus the oriT fragment was purified. This fragment was ligated to a purified BglII fragment from pHC79 which contains the cos site of lambda. Recombinant plasmids with the desired structure were obtained and one of these was named pRTP1. Further characterization of the restriction map of pRTP1 was performed and the map obtained is shown in Figure 3. There are unique sites for EcoRI, BamHi, HindIII, SalI, and BstEII.

c) Use of pRTP1 to return an unmarked ptx mutation.

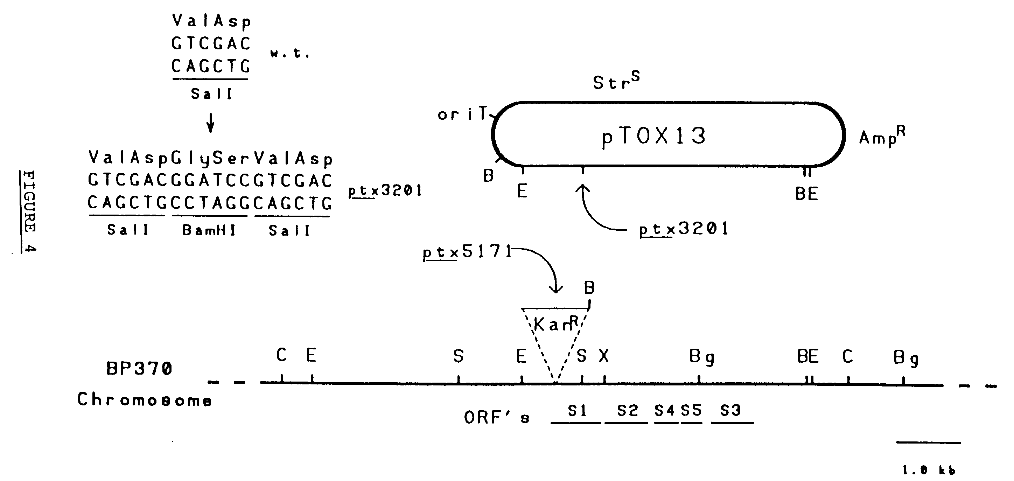

Figure 4 shows a map of pTOX13 which consists of pRTP1 into which has been cloned a 4.7 kb EcoRI fragment containing the structural genes for pertussis toxin. Assignment of the open reading frames coding for the subunits of this toxin S1, S2, S3, S4, and S5 is according to the DNA sequence of this region and by mutational analysis of this region. Shown in the figure is the position of mutation ptx3201. This mutation was constructed by ligating into the SalI site in the open reading frame of S1, after creating flush ends with DNA polymerase I, the DNA linker 5'-CGGATCCG-3'. The net effect, as shown in Figure 4 (upper left hand corner), is to introduce 12 bp causing a duplication of the Val-Asp codons and introducing between them codons for Gly-Ser in the primary sequence of the S1 subunit of pertussis toxin thus maintaining the open reading frame. In addition a BamHI site is introduced at this position.

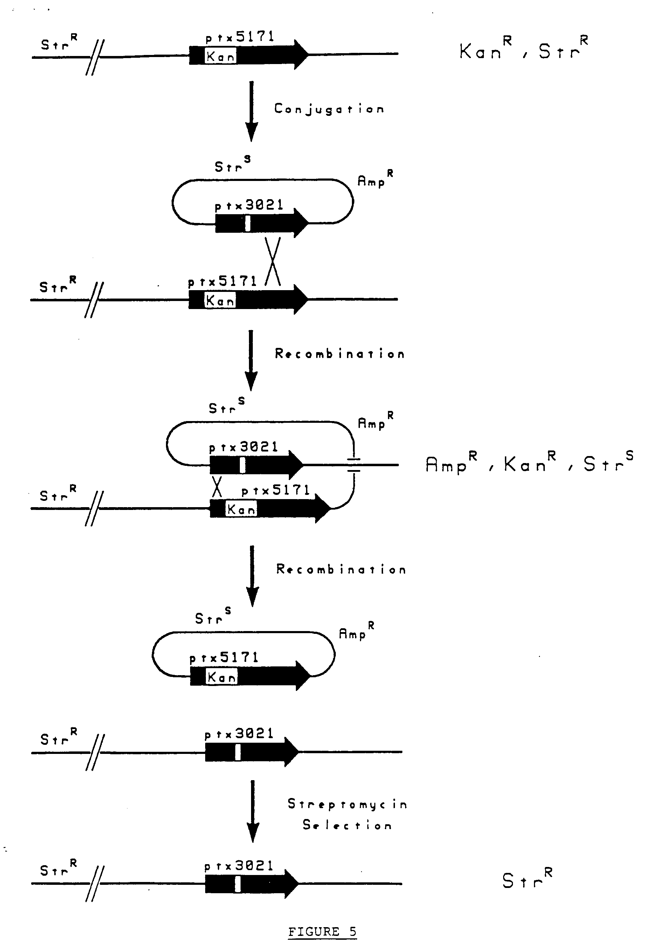

The recipient B. pertussis strain into which this mutation was introduced was BP370 ptx5171. This strain has an insertion of a kanamycin resistance module at the position shown in Figure 4. This module was introduced as a BamHI fragment into a Sau3A site in this region, explaining how a BamHI site is regenerated at one end of the insertion, but not the other, as shown. Thus it is possible to screen for replacement of this ptx allele with the 3201 allele by loss of kanamycin resistance. The steps in the exchange of these two alleles are diagrammed in Figure 5. HB101 containing pTOX13-ptx3201 was used in a triparental mating with HB101(pRKTV5) and BP370-ptx5171. Selection was for kanamycin and ampicillin resistance. Exconjugants arising from this mating were maintained on BG agar containing ampicillin. When streaked onto BG containing streptomycin, streptomycin resistant colonies were obtained and were tested

EP 0 275 689 B1

for resistance to kanamycin. Of approximately 200 colonies tested, four kanamycin sensitive candidates were obtained. This rather low frequency is presumably due to the fact that one region of flanking homology in which crossover must occur to result in allelic exchange is much shorter than the other (see Figure 4). Thus in most instances both crossover events which are selected in this process occur at the same side of the mutant alleles or between them such that exchange does not occur.

The introduction of ptx3201 was verified in the four candidates by southern hybridization. BamHI digested chromosomal DNA was blotted and probed with the EcoRI fragment of pTOX13. In the parental BP370 the toxin gene is found on a 15 kb BamHI fragment. The introduction of ptx5171 had introduced a BamHI site 4.1 kb from one end of this fragment, while in the four kanamycin sensitive candidates (lanes c-r), a BamHI site has been introduced 3.5 kb from one end, in keeping with the position of this mutation as shown in Figure 4. From this analysis it can be seen that all four candidates have the original insertion mutation removed and have gained a BamHI site at the correct location for the introduction of ptx3201.

All publications cited in this specification are illustrative of the skill of those skilled in the art to which this invention pertains. Each publication is individually herein incorporated by reference to the same extent as if each publication were individually incorporated by reference.

Invention now being fully described, it will be apparent to one or ordinary skill of the art that many changes and modifications can be made thereto.

## Claims

1. A nucleotide sequence comprising genetic information coding for the S1 subunit of pertussis toxin protein isolated from Bordetella pertussis, wherein the sequence has been modified to code for a mutated S1 subunit of pertussis toxin, where said mutated S1 subunit is capable of interaction with pertussis toxin B subunit to form a holotoxin, which holotoxin lacks the toxicity of wild-type pertussis toxin but has protective immunogenicity.

2. The sequence according to Claim 1 wherein 3x nucleotides are inserted into an S1-encoding segment of said toxin, wherein x is an integer of from 1 to 20.

3. The sequence according to Claim 2, wherein said nucleotides are inserted into the Sa1I site of the S1 subunit open reading frame.

4. The sequence according to Claim 2 or Claim 3 wherein the insertion comprises twelve nucleotides coding for the amino acids Val-Asp-Gly-Ser.

5. The sequence according to Claim 1 wherein the modified genetic information comprises one or more specific mutations in the sequence coding for the S1 subunit, wherein each such specific mutation results in a change in an amino acid.

6. A polypeptide consisting of a sequence of amino acids, wherein said polypeptide is a mutated S1 subunit encoded by the nucleotide sequence of any one of claims 1 to 5 which on interaction with pertussis toxin B subunit forms a holotoxin, which holotoxin lacks the toxicity of wild-type pertussis toxin but has protective immunogenicity.

7. A pertussis holotoxin comprising a B subunit of pertussis toxin and a mutated S1 subunit, which holotoxin lacks the toxicity of wild-type pertussis toxin but has protective immunogenicity.

8. A holotoxin according to Claim 7, wherein the mutated S1 subunit is according to Claim 6.

9. A holotoxin according to Claim 7 or Claim 8, wherein said mutated S1 subunit comprises ptx3201, as shown in Figure 4.

10. A method of producing a mutated S1 subunit of a pertussis holotoxin which is capable of interaction with pertussis B subunit to form a holotoxin, which holotoxin lacks the virulence of wild-type pertussis toxin but is capable of immunogenicity, said method comprising:
    culturing a Bordetella pertussis strain which has been modified to contain a tac promoter operably inserted upstream from said subunit gene whereby said subunit is constitutively expressed, and isolating said subunit from said culture medium.

16

**11.** The method of Claim 10, wherein said mutated S1 subunit has an insertion of from 1 to 20 amino acids.

**12.** The method of Claim 10 or Claim 11, wherein said mutated S1 subunit comprises ptx3201, as shown in Figure 4.

**Patentansprüche**

**1.** Genetische Information umfassende Nukleotidsequenz, die für die aus Bordetella pertussis isolierte S1-Untereinheit von Pertussistoxinprotein kodiert, worin die Sequenz modifiziert worden ist, um für eine mutierte S1-Untereinheit von Pertussistoxin zu kodieren, wobei die genannte mutierte S1-Untereinheit zur Interaktion mit Pertussistoxin B-Untereinheit fähig ist, um ein Holotoxin zu bilden, welchem Holotoxin die Toxizität von Wildtyp-Pertussistoxin fehlt, welches aber schützende Immunogenität aufweist.

**2.** Sequenz nach Anspruch 1, worin 3x Nukleotide in ein für S1 kodierendes Segment des genannten Toxins eingefügt sind, worin x eine ganze Zahl von 1 bis 20 ist.

**3.** Sequenz nach Anspruch 2, worin die genannten Nukleotide in die SalI-Stelle des offenen Leserasters der S1-Untereinheit eingefügt sind.

**4.** Sequenz nach Anspruch 2 oder 3, worin die Einfügung zwölf Nukleotide umfaßt, die für die Aminosäuren Val-Asp-Gly-Ser kodieren.

**5.** Sequenz nach Anspruch 1, worin die modifizierte genetische Information eine oder mehrere spezifische Mutationen in der für die S1-Untereinheit kodierenden Sequenz umfaßt, worin jede derartige spezifische Mutation zu einer Änderung in einer Aminosäure führt.

**6.** Polypeptid bestehend aus einer Sequenz von Aminosäuren, worin das genannte Polypeptid eine mutierte S1-Untereinheit ist, für welche die Nukleotidsequenz nach einem der Ansprüche 1 bis 5 kodiert, die bei der Interaktion mit Pertussistoxin B-Untereinheit ein Holotoxin bildet, welchem Holotoxin die Toxizität von Wildtyp-Pertussistoxin fehlt, welches aber schützende Immunogenität aufweist.

**7.** Pertussisholotoxin, umfassend eine B-Untereinheit von Pertussistoxin und eine mutierte S1-Untereinheit, welchem Holotoxin die Toxizität von Wildtyp-Pertussistoxin fehlt, welches aber schützende Immunogenität aufweist.

**8.** Holotoxin nach Anspruch 7, worin die mutierte S1-Untereinheit eine solche nach Anspruch 6 ist.

**9.** Holotoxin nach Anspruch 7 oder 8, worin die genannte mutierte S1-Untereinheit ptx3201 umfaßt, wie in Figur 4 gezeigt.

**10.** Verfahren zum Erzeugen einer mutierten S1-Untereinheit eines Pertussisholotoxins, das zur Interaktion mit Pertussis B-Unterheit fähig ist, um ein Holotoxin zu bilden, welchem Holotoxin die Virulenz von Wildtyp-Pertussistoxin fehlt, das aber zur Immunogenität fähig ist, wobei das genannte Verfahren umfaßt:
Kultivieren eines Bordetella pertussis-Stammes, der modifiziert wurde, um einen tac-Promotor zu enthalten, der operabel stromaufwärts vom genannten Untereinheitgen eingefügt ist, wodurch die genannte Untereinheit konstitutiv exprimiert wird, und Isolieren der genannten Untereinheit vom genannten Kulturmedium.

**11.** Verfahren nach Ansprch 10, worin die genannte mutierte S1-Untereinheit eine Einfügung von 1 bis 20 Aminosäuren aufweist.

**12.** Verfahren nach Anspruch 10 oder 11, worin die genannte mutierte S1-Untereinheit ptx3201 umfaßt, wie in Figur 4 gezeigt.

**Revendications**

17

1. Séquence de nucléotides comprenant une information génétique codant pour la sous-unité S1 de la protéine toxine du pertussis isolée de Bordetella pertussis, où la séquence a été modifiée pour coder pour une sous-unité S1 mutée de la toxine du pertussis, où ladite sous-unité S1 mutée est capable d'une interaction avec la sous-unité B de la toxine du pertussis pour former une holotoxine, laquelle holotoxine manque de la toxicité de la toxine du pertussis du type sauvage mais a une immunogénicité protectrice.

2. Séquence selon la revendication 1, où l'on insère 3x nucléotides dans un segment de codage de S1 de ladite toxine, où x est un nombre entier de 1 à 20.

3. Séquence selon la revendication 2, où lesdits nucléotides sont insérés dans le site SalI du cadre de lecture ouverte de la sous-unité S1.

4. Séquence selon la revendication 2 ou la revendication 3, où l'insertion comprend douze nucléotides codant pour les acides aminés Val-Asp-Gly-Ser.

5. Séquence selon la revendication 1, où l'information génétique modifiée comprend une ou plusieurs mutations spécifiques dans la séquence codant pour la sous-unité S1, où chaque mutation spécifique a pour résultat un changement d'un acide aminé.

6. Polypeptide consistant en une séquence d'acides aminés, où ledit polypeptide est une sous-unité S1 mutée codée par une séquence de nucléotides selon l'une quelconque des revendications 1 à 5 qui, lors d'une interaction avec la sous-unités B de la toxine du pertussis, forme une holotoxine, laquelle holotoxine manque de la toxicité de la toxine du pertussis du type sauvage mais a une immunogénicité protectrice.

7. Holotoxine du pertussis comprenant une sous-unité B de la toxine du pertussis et une sous-unité S1 mutée, laquelle holotoxine manque de la toxicité de la toxine du pertussis du type sauvage mais a une immunogénicité protectrice.

8. Holotoxine selon la revendication 7, où la sous-unité S1 mutée est selon la revendication 6.

9. Holotoxine selon la revendication 7 ou la revendication 8, où ladite sous-unité S1 mutée comprend ptx3201, comme le montre la figure 4.

10. Méthode de production d'une sous-unité S1 mutée d'une holotoxine du pertussis qui est capable d'une interaction avec la sous-unité B du pertussis pour former une holotoxine, laquelle holotoxine manque de la virulence de la toxine du pertussis du type sauvage mais est capable d'une immunogénicité, ladite méthode comprenant:
   - la mise en culture d'une souche de Bordetella pertussis qui a été modifiée pour contenir un promoteur tac activement inséré en amont dudit gène de la sous-unité pour qu'ainsi ladite sous-unité puisse s'exprimer constitutivement et l'isolement de ladite sous-unité dudit milieu de culture.

11. Méthode de la revendication 10, où ladite sous-unité S1 mutée a une insertion de 1 à 20 acides aminés.

12. Méthode de la revendication 10 ou de la revendication 11, où ladite sous-unité S1 mutée comprend ptx3201, comme le montre la figure 4.

FIGURE 1

19

FIGURE 2

20

FIGURE 3

VaIAsp
GTCGAC w.t.
CAGCTG
Sall

↓

VaIAspGIySerVaIAsp
GTCGACGGATCCGTCGAC ptx3201
CAGCTGCCTAGGCAGCTG
Sall    BamHI    Sall

FIGURE 4

Str$^S$

oriT

pTOX13

Amp$^R$

B

E                                    BE

ptx3201

ptx5171

B

Kan$^R$

BP370    C E        S     E   S X      Bg      BE C   Bg

Chromosome

ORF's    S1   S2  S4 S5  S3

1.0 kb

FIGURE 5

23